(19) Europäisches Patentamt
European Patent Office
Office européen des brevets

(11) Veröffentlichungsnummer: **0 320 438 B1**

# (12) EUROPÄISCHE PATENTSCHRIFT

(45) Veröffentlichungstag der Patentschrift: **23.09.92**

(51) Int. Cl.[5]: **C07J 13/00**, A61K 31/56

(21) Anmeldenummer: **88730274.3**

(22) Anmeldetag: **05.12.88**

## (54) 17-Halogenmethylen-estratriene.

(30) Priorität: **07.12.87 DE 3741800**

(43) Veröffentlichungstag der Anmeldung:
**14.06.89 Patentblatt 89/24**

(45) Bekanntmachung des Hinweises auf die Patenterteilung:
**23.09.92 Patentblatt 92/39**

(84) Benannte Vertragsstaaten:
**AT BE CH DE ES FR GB GR IT LI LU NL SE**

(56) Entgegenhaltungen:
**US-A- 3 536 703**
**US-A- 3 716 530**

**CHEMICAL ABSTRACTS, Band 72, Nr. 23, 8. Juni 1970, Nr. 121098h, Columbus, Ohio, US; M. SCHLOSSER et al.: "Fluoro olefins by fluoromethylenation of carbonyl compounds", & SYNTHESIS 1969, (2), 75-6**

(73) Patentinhaber: **SCHERING AKTIENGESELLSCHAFT Berlin und Bergkamen**
**Müllerstrasse 170/178 Postfach 65 03 11**
**W-1000 Berlin 65(DE)**

(72) Erfinder: **Jungblut, Peter, Prof. Dr.**
**Kampweg 4**
**W-3057 Neustadt-Büren(DE)**
Erfinder: **Wiechert, Rudolf, Prof. Dr.**
**Petzower Strasse 8A**
**W-1000 Berlin 39(DE)**
Erfinder: **Bohlmann, Rolf, Dr.**
**Melanchtonstrasse 23**
**W-1000 Berlin 21(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die Erfindung betrifft neue 17-Halogenmethylen-estratriene, Verfahren zu deren Herstellung und diese enthaltende pharmazeutische Präparate.

Die neuen 17-Halogenmethylen-estratriene werden durch die allgemeine Formel I gekennzeichnet

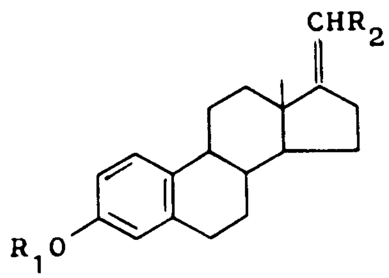

(I),

worin

$R_1$ ein Wasserstoffatom, eine Methyl- oder Acylgruppe und

$R_2$ ein Halogenatom

bedeuten.

Als Acylgruppen kommen physiologisch verträgliche Gruppen infrage, die sich von Säuren ableiten, die üblicherweise zur Veresterung von Hydroxysteroiden verwendet werden. Hierzu zählen insbesondere organische Carbonsäuren mit 1 bis 12 Kohlenstoffatomen, die der aliphatischen, cycloaliphatischen, aromatischen, aromatisch-aliphatischen oder heterocyclischen Reihe angehören und die gesättigt oder ungesättigt, ein- oder mehrbasisch und/oder substituiert sein können. Als Beispiele für die Substituenten seien Alkyl-, Hydroxy-, Alkoxy-, Oxo- oder Aminogruppen und Halogenatome erwähnt. Hierzu zählen auch die gebräuchlichen anorganischen Säuren.

Als Carbonsäuren mit 1 bis 12 Kohlenstoffatomen seien beispielsweise genannt: Ameisensäure, Essigsäure, Propionsäure, Buttersäure, Isobuttersäure, Valeriansäure, Isovaleriansäure, Capronsäure, Önanthsäure, Caprylsäure, Pelargonsäure, Caprinsäure, Undecylsäure, Laurinsäure, Trimethylessigsäure, tert. Butylessigsäure, Cyclopentylessigsäure, Diäthylaminoessigsäure, Morpholinoessigsäure, Milchsäure, Bernsteinsäure, Adipinsäure, Benzoesäure, Nikotinsäure u.a.

Als anorganische Säuren kommen zum Beispiel Schwefel- und Phosphorsäure in Betracht.

Die Ester der Bernsteinsäure, Adipinsäure, Schwefelsäure und Phosphorsäure können gegebenenfalls mit Alkali in die wasserlöslichen Salze überführt werden.

Als Halogenatome kommen Fluor, Chlor, Brom und Jod. vorzugsweise Fluor und Chlor, infrage.

Es wurde nun gefunden, daß die in 17-Stellung durch Halogenmethylen substituierten Estratriene sich deutlich von den Estronen unterscheiden. Im Vergleich zu den Estronen, aus denen sie hergestellt werden, zeigen die Verbindungen der allgemeinen Formel I eine geringere Affinität zu den Östrogenrezeptoren als Estradiol und bewirken eine im Vergleich mit Estradiol erhöhte Zellmembran- und Blut-/Lymphgefäßpermeabilität.

Im Östrogen-Rezeptor-Bindungstest auf östrogene Wirkung unter Verwendung von Zytosol aus Schweineuterushomogenat und von 6,7$^3$H-Estradiol als Bezugssubstanz zeigen die Verbindungen der allgemeinen Formel I eine geringe Affinität zum Östrogenrezeptor.

In der folgenden Tabelle wird die Kompetition am Rezeptor in Prozent angegeben.

Tabelle

| Östrogen-Rezeptor-Bindungstest | | |
|---|---|---|
| Verbindung | Mol | % Kompetition |
| Estradiol | $2x10^{-8}$<br>$2x10^{-7}$<br>$2x10^{-6}$ | 49<br>88<br>96 |
| 17-Fluormethylen-1,3,5(10)-estratrien-3-ol | $2x10^{-8}$<br>$2x10^{-7}$<br>$2x10^{-6}$ | 12<br>23<br>78 |

Im Unteruswachstumstest mit infantilen, 23 Tage alten Spraque-Dawley Ratten hat zum Beispiel 17-Fluormethylen-1,3,5(10)-estratrien-3-ol 1/40 der uterotropen Aktivität von Estradiol, wenn Uterusfeuchtgewichte einschließlich der Intrauterinflüssigkeit zugrundegelegt werden. Wird dagegen der DNA-Gehalt als Maß der Uteruszellzahl genommen, ergibt sich für 17-Fluormethylen-1,3,5(10)-estratrien etwa 1/70 der Estradiolwirkung.

Zur Ausführung des Tests erhalten die infantilen weiblichen Ratten einmal täglich über 3 Tage subcutan Estradiol oder 17-Fluormethylen-1,3,5(10)-estratrien-3-ol. Am 4. Tag werden die Tiere getötet und das Uterusgewicht oder der DNA-Gehalt pro Uterus bestimmt.

Ein Uterusgewicht von 67 mg wird mit 0.1 µg Estradiol oder mit 4,2 µg 17-Fluormethylen-1,3,5(10)-estratrien-3-ol erzielt. Ein DNA-Gehalt von 318 µg wird mit 0.1 µg Estradiol oder mit 7,3 µg 17-Fluormethylen-1,3,5(10)-estratrien-3-ol erreicht.

Nach lokaler Applikation von Estradiol oder 17-Fluormethylen-1,3,5(10)-estratrien-3-ol in das Uteruslumen des Schweins wird ein Uterusödem erzeugt, das bei der 17-Fluormethylenverbindung stärker ausgeprägt ist als bei Estradiol. Das Ausmaß des Ödems kann durch Bestimmung des Albumin- und DNA-Gehalts ermittelt werden.

Die intrauterine Injektion von $1x10^{-6}$ molaren Lösungen (20-50 ml/Uterus) der zu testenden Verbindungen führt nach 120 Minuten bei Estradiol zu einem Anstieg des Albumingehalts auf ca. 17 mg Albumin/1 mg DNA und bei der entsprechenden 17-Fluormethylenverbindung zu einem Anstieg auf 36 mg Albumin/1 mg DNA.

Das Einbringen der Testsubstanz in ein Uterushorn des Schweins bewirkt nur dort eine Ödembildung; das unbehandelte Horn wird nicht beeinflußt. Die Substanz führt zu einem Verbrauch an Rezeptor ohne nachfolgende Neusynthese.

Für die Verbindungen der allgemeinen Formel I findet man demnach eine Wirkungsdisproportionierung im Sinne einer geringeren Wirkung im Zellkern über den Östrogenrezeptor bei im Vergleich zum Estradiol unveränderter Ödembildung.

Als Substrate für intrazelluläre Enzyme, deren Produkte zu einer Erhöhung der Zellmembran- und Blut-/Lymphgefäßpermeabilität führen, die sich als sogenannte "Wasserimbibition" in Form eines massiven Ödems im Zielorgan Uterus demonstrieren läßt, sind die Verbindungen der allgemeinen Formel I besonders geeignet zur Behebung klimakterischer Beschwerden sowie allgemein zur Behandlung von Erscheinungen, die durch Ausfall des zweiten Wirkungsabschnitts von Estradiol auftreten.

Die Wirkstoffe werden vorzugsweise oral appliziert, sie können jedoch auch lokal und parenteral verabreicht werden. Dazu werden die Wirkstoffe mit den in der galenischen Pharmazie üblichen Zusätzen, Trägerstoffen und/oder Lösungsvermittlern nach an sich bekannten Methoden zu den üblichen Applikationsformen verarbeitet. Für die bevorzugte orale Applikation kommen insbesondere Tabletten, Dragees, Kapseln, Pillen, wäßrige Suspensionen oder alkoholische Lösungen infrage und für die lokale und parenterale Applikation insbesondere Salben bzw. ölige Lösungen, wie zum Beispiel Sesam- oder Rizinusöllösungen, die gegebenenfalls zusätzlich einen Lösungsvermittler, wie zum Beispiel Benzylbenzoat, enthalten können.

Die Wirkstoffkonzentration in den pharmazeutischen Zubereitungen ist abhängig von der Applikationsform und vom Anwendungsgebiet. So können zum Beispiel Tabletten, Dragees, Kapseln oder Pillen 10 - 150 µg Wirkstoff pro Dosiseinheit und ölige Lösungen oder Salben 1 - 20 µg Wirkstoff pro ml enthalten.

In einer bevorzugten Ausführungsform enthält die orale Applikationsform 10 bis 100 µg Wirkstoff.

Nach einer Behandlung von therapeutisch kastrierten Frauen sowie von Frauen im Klimakterium, die jeweils unter Hitzewallungen und Niedergeschlagenheit litten, mit täglich 10 bis 100 µg Wirkstoff der allgemeinen Formel I trat bereits nach 2 Tagen eine deutliche Besserung der Beschwerden auf.

Die systemische Gabe von Verbindungen der allgemeinen Formel I an Spraque-Dawley Ratten mit

durch 7,12-Dimethylbenzanthracen induzierten Mammatumoren führt zu einem Sistieren des Tumorwachstums ohne merkliche Beeinflussung des Zyklus. Die Verbindungen sind damit auch zur Behandlung von hormonabhängigen Tumoren geeignet. Durch Anwendung der Verbindungen in täglichen Mengen von 0,1 - 5 µg pro kg wird die Wachstumsförderung bestehender hormonabhängiger Tumore unterbunden. Die Antitumorwirkung wird in an sich bekannter Weise bestimmt, zum Beispiel wie in Science 137 (1962) 257-262 beschrieben.

Als Substanzen mit selektiver östrogener Wirkung können die Verbindungen der allgemeinen Formel I auch in Präparaten zur Kontrazeption verwendet werden, vorzugsweise in Kombination mit einer gestagen wirksamen Hormonkomponente, wie zum Beispiel Levonorgestrel, Gestoden oder Desogestrel. Oral applizierbare Applikationsformen enthalten vorzugsweise 10 - 100 µg einer Verbindung der allgemeinen Formel I und 50 - 500 µg eines stark wirksamen Gestagens.

Die neuen 17-Halogenmethylen-estratriene der allgemeinen Formel I werden erfindungsgemäß dadurch hergestellt, daß man ein Östron der allgemeinen Formel II

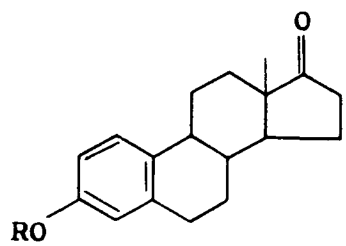

(II),

worin

R ein Wasserstoffatom oder eine Methylgruppe bedeutet,

mit einem Halogenmethylenylid umsetzt und gegebenenfalls eine freie Hydroxygruppe acyliert.

Die Umsetzung mit Halogenmethylenylid erfolgt nach an sich bekannten Methoden, beispielsweise in einem aprotischen Lösungsmittel wie Dimethylsulfoxid, Dimethylformamid, Dioxan, Tetrahydrofuran oder einem Gemisch dieser Lösungsmittel bei Temperaturen zwischen 20 und 40 °C, wobei zweckmäßigerweise unter Schutzgas, wie Stickstoff oder Argon, gearbeitet wird (Pure and Applied Chemistry 1980, Band 52, Seite 771).

Die Herstellung des Halogenmethylenylids erfolgt zweckmäßigerweise in der Reaktionslösung aus Halomethyltriphenylphosphoniumsalz mit einer Base, wie Natriumhydrid, Natriumhydroxid, Kalium-tertiärbutylat, Natriummethylat oder Butyllithium (Journal Fluorine Chemistry 1985, Band 27, Seite 85).

Als Halomethyltriphenylphosphoniumsalze kommen insbesondere Fluormethyltriphenylphosphoniumtetrafluorborat und Chlormethyltriphenylphosphoniumchlorid infrage.

Die sich gegebenenfalls anschließende Acylierung erfolgt nach bekannten Methoden zur Veresterung phenolischer Hydroxygruppen, vorzugsweise mit Pyridin/Säureanhydrid oder Pyridin/Säurechlorid bei Raumtemperatur [Ang. Chemie 90 (1978) Seite 602].

Beispiel 1

17,2 g Fluormethyltriphenylphosphoniumtetrafluorborat [J. Fluorine Chem. 27 (1985) 85-89] werden in 150 ml Dioxan suspendiert und bei 20 °C mit 6,7 g Kaliumtertiärbutylat portionsweise versetzt und 0,5 Stunden nachgerührt. Zu dieser Lösung werden 2,0 g 1,3,5(10)-Estratrien-3-ol-17-on in 30 ml Dioxan gegeben und 30 Minuten gerührt. Zur Aufarbeitung wird auf Wasser gegeben, über Natriumsulfat getrocknet und im Vakuum zur Trockne eingeengt. Nach der chromatographischen Reinigung an Kieselgel mit Hexan/Essigester erhält man 1,63 g 17-Fluormethylen-1,3,5(10)-estratrien-3-ol als E/Z-Gemisch vom Schmelzpunkt 125 -129 °C, $[\alpha]_D$ +68,1 ° (Chloroform).

Beispiel 2

17,2 g Chlormethyltriphenylphosphoniumchlorid werden in 150 ml Dioxan suspendiert und bei 20 °C mit 6,7 g Kaliumtertiärbutylat portionsweise versetzt und 0,5 Stunden nachgerührt. Zu dieser Lösung werden 2 g 3-[(Tetrahydropyran-2-yl)oxy]-1,3,5(10)-estratrien-17-on in 30 ml Dioxan gegeben und 30 Minuten gerührt. Zur Aufarbeitung wird auf Wasser gegeben, über Natriumsulfat getrocknet und im Vakuum zur Trockne eingeengt. Man erhält 17-Chlormethylen-3-[(tetrahydropyran-2-yl)oxy]-1,3,5(10)-estratrien, das

als Rohprodukt in 50 ml Methanol gelöst und 1 Stunde mit 2 g Oxalsäure unter Rückfluß erhitzt wird. Dann wird mit Eiswasser/Kochsalz gefällt, mit Essigester aufgenommen, über Natriumsulfat getrocknet und im Vakuum zur Trockne eingeengt. Nach der chromatographischen Reinigung an Kieselgel mit Hexan/Essigester erhält man 1,74 g 17-Chlormethylen-1,3,5(10)-estratrien-3-ol als E/Z-Gemisch vom Schmelzpunkt 130 - 133 °C $[\alpha]_D$ +52,8° (Chloroform).

Beispiel 3

Analog Beispiel 1 werden 2,0 g 3-Methoxyestron zu 1,67 g 17-Fluormethylen-3-methoxy-1,3,5(10)-estratrien als Öl umgesetzt.

Beispiel 4

1,0 g 17-Fluormethylen-1,3,5(10)-estratrien-3-ol in 10 ml Pyridin werden mit 5 ml Acetanhydrid 2 Stunden bei 20 °C gerührt. Dann wird mit schwefelsaurem Eiswasser gefällt, in Dichlormethan aufgenommen, mit Natriumhydrogencarbonatlösung neutral gewaschen, über Magnesiumsulfat getrocknet und im Vakuum vom Lösungsmittel befreit. Nach Chromatographie an Kieselgel mit Hexan/Essigester erhält man 955 mg 3-Acetoxy-17-fluormethylen-1,3,5(10)-estratrien.

Beispiel 5

Analog Beispiel 4 werden 1,0 g 17-Fluormethylen-1,3,5(10)-estratrien-3-olin 5 ml Buttersäureanhydrid zu 1,03 g 3-Butyryloxy-17-fluormethylen-1,3,5(10)-estratrien umgesetzt.

Beispiel 6

Analog Beispiel 4 werden 1,0 g 17-Fluormethylen-1,3,5(10)-estratrien-3-olin 5 ml Undecylsäureanhydrid zu 1,06 g 17-fluormethylen-3-undecyloxy-1,3,5(10)-estratrien umgesetzt.

Beispiel 7

Analog Beispiel 2 werden 2,0 g 3-Methoxyestron zu 1,59 g 17-Chlormethylen-3-methoxy-1,3,5(10)-estratrien als Öl umgesetzt.

Beispiel 8

1,0 g 17-Chlormethylen-1,3,5(10)-estratrien-3-ol in 10 ml Pyridin werden mit 5 ml Acetanhydrid 2 Stunden bei 20 °C gerührt. Dann wird mit schwefelsaurem Eiswasser gefällt, mit Dichlormethan aufgenommen, mit Natriumhydrogencarbonatlösung neutral gewaschen, über Magensiumsulfat getrocknet und im Vakuum vom Lösungsmittel befreit. Nach Chromatographie an Kieselgel mit Hexan/Essigester erhält man 955 mg 3-Acetoxy-17-chlormethylen-1,3,5(10)-estratrien.

Beispiel 9

Analog Beispiel 8 werden 1,0 g 17-Chlormethylen-1,3,5(10)-estratrien-3-olin 5 ml Buttersäureanhydrid zu 1,03 g 3-Butyryloxy-17-chlormethylen-1,3,5(10)-estratrien umgesetzt.

Beispiel 10

Analog Beispiel 8 werden 1,0 g 17-Chlormethylen-1,3,5(10)-estratrien-3-olin 5 ml Undecylsäureanhydrid zu 1,06 g 17-Chlormethylen-3-undecyloxy-1,3,5(10)-estratrien umgesetzt.

Beispiel 11

**Zusammensetzung eines Dragees**

| | |
|---|---|
| 0,010 mg | 17-Fluormethylen-1,3,5(10)-estratrien-3-ol |
| 46,490 mg | Lactose |
| 26,800 mg | Maisstärke |
| 3,000 mg | Poly(1-vinyl-2-pyrrolidon) Mittleres MG 25 000 |
| 3,700 mg | Talkum |
| 80,000 mg | Gesamtgewicht, das mit üblicher Zuckermischung auf etwa 140 mg ergänzt wird. |

Beispiel 12

**Zusammensetzung einer alkoholischen Lösung**

1 mg 17-Fluormethylen-1,3,5(10)-estratrien -3-ol werden in 10 ml 46 %igen Ethylalkohol gelöst. In zehn Tropfen (0,5 ml) sind 50 µg Wirkstoff enthalten.

**Patentansprüche**
**Patentansprüche für folgende Vertragsstaaten : AT, BE, CH, DE, FR, GB, IT, LI, LU, NL, SE**

**1.** 17-Halogenmethylen-estratriene der allgemeinen Formel I

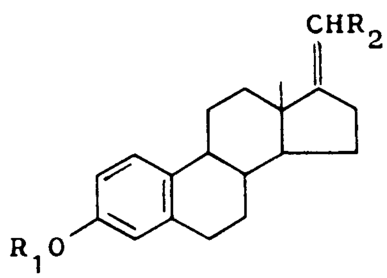

(I),

worin

$R_1$ ein Wasserstoffatom, eine Methyl- oder Acylgruppe und
$R_2$ ein Halogenatom

bedeuten.

**2.** 17-Fluormethylen-1,3,5(10)-estratrien-3-ol,
17-Fluormethylen-3-methoxy-1,3,5(10)-estratrien,
3-Acetoxy-17-fluormethylen-1,3,5(10)-estratrien,
3-Butyryloxy-17-fluormethylen-1,3,5(10)-estratrien und
17-Fluormethylen-3-undecyloxy-1,3,5(10)-estratrien.

**3.** 17-Chlormethylen-1,3,5(10)-estratrien-3-ol,
17-Chlormethylen-3-methoxy-1,3,5(10)-estratrien,
3-Acetoxy-17-chlormethylen-1,3,5(10)-estratrien,
3-Butyryloxy-17-chlormethylen-1,3,5(10)-estratrien und
17-Chlormethylen-3-undecyloxy-1,35(10)-estratrien.

**4.** Pharmazeutische Präparate, gekennzeichnet durch den Gehalt an einer oder mehreren Verbindung(en) gemäß Anspruch 1 bis 3.

**5.** Verwendung von Verbindungen gemäß Anspruch 1 - 3 zur Herstellung von Präparaten zur Behandlung von Östrogenmangelerscheinungen.

**6.** Verfahren zur Herstellung von 17-Halogenmethylen-estratrienen der allgemeinen Formel I

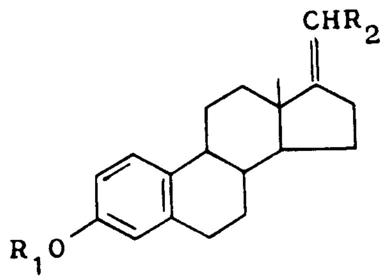

(I),

worin

$R_1$ ein Wasserstoffatom, eine Methyl- oder Acylgruppe und

$R_2$ ein Halogenatom

bedeuten,

dadurch gekennzeichnet, daß man ein Östrogen der allgemeinen Formel II

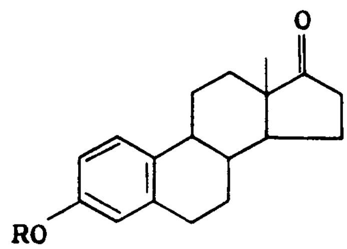

(II),

worin

R ein Wasserstoffatom oder eine Methylgruppe bedeutet, mit einem Halogenmethylenylid umsetzt und gegebenenfalls eine freie 3-Hydroxygruppe acyliert.

**Patentanspruch für folgende Vertragsstaaten : ES, GR**

1. Verfahren zur Herstellung von 17-Halogenmethylen-estratriene der allgemeinen Formel I

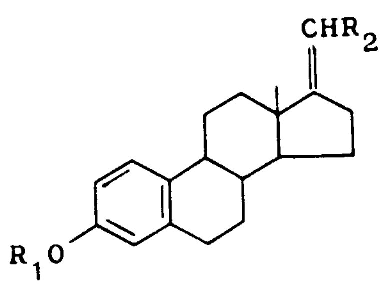

(I),

worin

$R_1$ ein Wasserstoffatom, eine Methyl- oder Acylgruppe und

$R_2$ ein Halogenatom

bedeuten,

dadurch gekennzeichnet, daß man ein Östrogen der allgemeinen Formel II

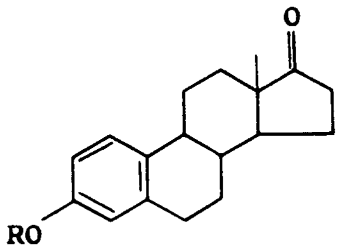

(II),

worin
R ein Wasserstoffatom oder eine Methylgruppe bedeutet, mit einem Halogenmethylenylid umsetzt und gegebenenfalls eine freie 3-Hydroxygruppe acyliert.

## Claims

**Claims for the following Contracting States : AT, BE, CH, DE, FR, GB, IT, LI, LU, NL, SE**

**1.** 17-Halomethylene-oestratrienes of the general formula I

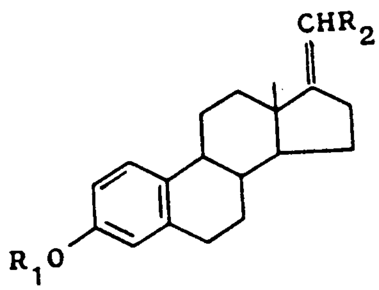

(I)

wherein
$R_1$ represents a hydrogen atom or a methyl or acyl group, and
$R_2$ represents a halogen atom.

**2.** 17-Fluoromethylene-1,3,5(10)-oestratrien-3-ol,
17-fluoromethylene-3-methoxy-1,3,5(10)-oestratriene,
3-acetoxy-17-fluoromethylene-1,3,5(10)-oestratriene,
3-butyryloxy-17-fluoromethylene-1,3,5(10)-oestratriene and
17-fluoromethylene-3-undecyloxy-1,3,5(10)-oestratriene.

**3.** 17-Chloromethylene-1,3,5(10)-oestratrien-3-ol,
17-chloromethylene-3-methoxy-1,3,5(10)-oestratriene,
3-acetoxy-17-chloromethylene-1,3,5(10)-oestratriene,
3-butyryloxy-17-chloromethylene-1,3,5(10)-oestratriene and
17-chloromethylene-3-undecyloxy-1,3,5(10)-oestratriene.

**4.** Pharmaceutical compositions, characterised in that they contain one or more compound(s) according to claims 1 to 3.

**5.** The use of compounds according to claims 1 to 3 for the manufacture of compositions for the treatment of oestrogen deficiency symptoms.

**6.** Process for the manufacture of 17-halomethylene-oestratrienes of the general formula I

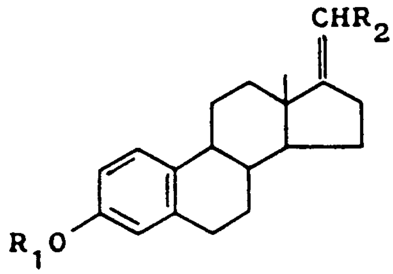

(I)

wherein
$R_1$ represents a hydrogen atom or a methyl or acyl group, and
$R_2$ represents a halogen atom,
characterised in that an oestrogen of the general formula II

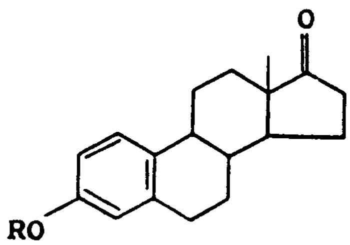

(II)

wherein
R represents a hydrogen atom or a methyl group, is reacted with a halomethylene ylide and optionally a free 3-hydroxy group is acylated.

**Claim for the following Contracting States : ES, GR**

**1.** Process for the manufacture of 17-halomethylene-oestratrienes of the general formula I

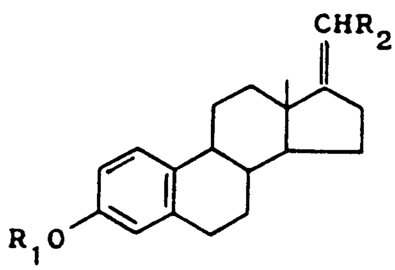

(I)

wherein
$R_1$ represents a hydrogen atom or a methyl or acyl group, and
$R_2$ represents a halogen atom,
characterised in that an oestrogen of the general formula II

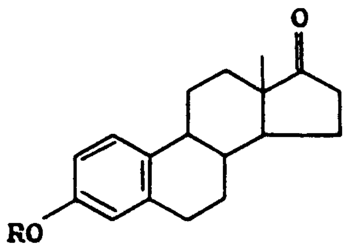

(II)

wherein
R represents a hydrogen atom or a methyl group, is reacted with a halomethylene ylide and optionally a free 3-hydroxy group is acylated.

## Revendications

**Revendications pour les Etats contractants suivants : AT, BE, CH, DE, FR, GB, IT, LI, LU, NL, SE**

**1.** 17-Halogénométhylène-estratriènes répondant à la formule générale I :

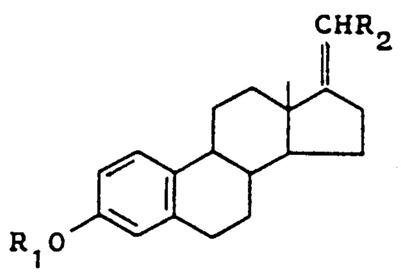

(I)

dans laquelle
$R_1$ représente un atome d'hydrogène, un radical méthyle ou un radical acyle et
$R_2$ représente un atome d'halogène.

**2.** 17-Fluorométhylène-1,3,5(10)-estratriène-3-ol,
17-Fluorométhylène-3-méthoxy-1,3,5(10)-estratriène,
3-Acétoxy-17-fluorométhylène-1,3,5(10)-estratriène,
3-Butyryloxy-17-fluorométhylène-1,3,5(10)-estratriène et
17-Fluorométhylène-3-undécyloxy-1,3,5(10)-estratriène.

**3.** 17-Chlorométhylène-1,3,5(10)-estratriène-3-ol,
17-Chlorométhylène-3-méthoxy-1,3,5(10)-estratriène,
3-Acétoxy-17-chlorométhylène-1,3,5(10)-estratriène,
3-Butyryloxy-17-chlorométhylène-1,3,5(10)-estratriène et
17-Chlorométhylène-3-undécyloxy-1,3,5(10)-estratriène.

**4.** Compositions pharmaceutiques caractérisées en ce qu'elles contiennent un ou plusieurs composés selon l'une quelconque des revendications 1 à 3.

**5.** Application de composés selon l'une quelconque des revendications 1 à 3 à la fabrication de compositions destinées au traitement de symptômes d'oestrogénopénie.

**6.** Procédé pour préparer des 17-halogénométhylène-estratriènes répondant à la formule générale I :

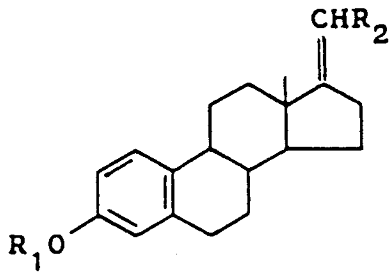

(I)

dans laquelle

$R_1$ représente un atome d'hydrogène, un radical méthyle ou un radical acyle et

$R_2$ représente un atome d'halogène,

procédé caractérisé en ce qu'on fait réagir un oestrogène répondant à la formule générale II :

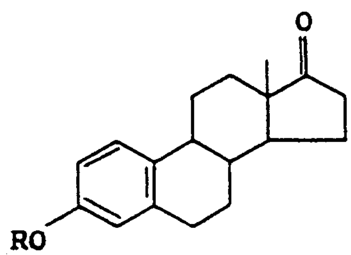

(II)

dans laquelle R représente un atome d'hydrogène ou un radical méthyle, avec un halogénométhylénylure, et on acyle éventuellement un radical hydroxy libre à la position 3.

**Revendication pour les Etats contractants suivants : ES, GR**

1. Procédé pour préparer des 17-halogénométhylène-estratriènes répondant à la formule générale I :

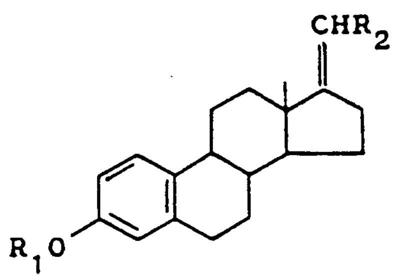

(I)

dans laquelle

$R_1$ représente un atome d'hydrogène, un radical méthyle ou un radical acyle et

$R_2$ représente un atome d'halogène,

procédé caractérisé en ce qu'on fait réagir un oestrogène répondant à la formule générale II :

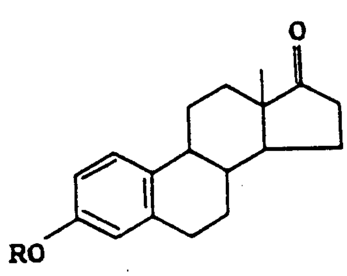

(II),

dans laquelle R représente un atome d'hydrogène ou un radical méthyle, avec un halogénométhylénylure, et on acyle éventuellement un radical hydroxy libre à la position 3.